# EUROPEAN PATENT APPLICATION

(11) **EP 4 020 053 A1**
(43) Date of publication of application: **29.06.2022**
(21) Application number: 21216679.7
(22) Date of filing: 21.12.2021
(51) Int. Cl.: G02B 21/10

(54) **CULTIVATION CONTAINER AND OBSERVATION SYSTEM**

(30) Priority: 25.12.2020 JP 2020216121
(71) Applicant: Yokogawa Electric Corporation, Tokyo 180-8750 (JP)
(72) Inventor: AOKI, Hidetoshi, Musashino-shi (JP)
(74) Representative: Osha Liang

(57) **Abstract**

A cultivation container (1) includes a waveguide substrate (2) configured to totally reflect and guide measurement light (L) incident from a side end surface (2cl) of the waveguide substrate (2), and a surrounding wall (3) standing upright on a top surface (2a) of the waveguide substrate (2) and forming a cell cultivation space (K). The surrounding wall may include a shielding part (3b) configured to shield the measurement light.

## Description

### BACKGROUND

### Technical Field

The present invention relates to a cultivation container and an observation system.

Priority is claimed on Japanese Patent Application No. 2020-216121, filed on December 25, 2020.

### Related Art

As a basic observation method of observing a specimen, bright field observation and dark field observation are exemplified. The bright field observation is a method of irradiating a specimen with uniform illumination light (measurement light) and observing the light passing through the specimen. On the other hand, the dark field observation is a method of irradiating a specimen with measurement light in an oblique direction and observing scattered light or reflected light generated by the specimen. When the bright field observation is performed on an adherent cell (a cell that proliferates while adhering to a cultivation container) during cultivation in the cultivation container, a contrast becomes low. For this reason, when the adherent cell is observed, the dark field observation may be performed. Further, Japanese Unexamined Patent Application Publication No. 2011-248216 and Japanese Unexamined Patent Application Publication No. 2018-081134 disclose a dark field optical system that enables dark field observation.

Incidentally, for example, when the dark field observation of the cell in the cultivation container is performed using the dark field optical system disclosed in Japanese Unexamined Patent Application Publication No. 2011-248216 and Japanese Unexamined Patent Application Publication No. 2018-081134, measurement light incident on the cultivation container in an oblique direction is transferred into the cultivation container and spreads over the entire cultivation container. As a result, measurement light may leak from various areas of the cultivation container and such leakage light may interfere with observation.

### SUMMARY

According to one aspect, the present disclosure relates to a cultivation container comprising a waveguide substrate configured to totally reflect and guide measurement light incident from a side end surface of the waveguide substrate, and a surrounding wall standing upright on a top surface of the waveguide substrate and forming a cell cultivation space.

Further embodiments of the cultivation container are defined in the dependent claims 2 to 9

According to a further aspect, the present disclosure relates toan observation system comprising the cultivation container as defined in any of the embodiments disclosed herein, and a light source configured to emit the measurement light incident on the waveguide substrate of the cultivation container.

Further embodiments of the observation system are defined in the dependent claims 11 to 20.

Further features and aspects of the present disclosure will become apparent from the following detailed description of exemplary embodiments with reference to the attached drawings.

### BRIEF DESCRIPTION OF THE DRAWINGS

FIG. 1 is a perspective view of a cultivation container according to a first embodiment.
FIG. 2A is a plan view of the cultivation container of the first embodiment.
FIG. 2B is a cross-sectional view along line A-A of FIG. 2A.
FIG. 3 is a schematic view of a case in which measurement light is incident into the cultivation container of the first embodiment.
FIG. 4A is a plan view of a cultivation container according to a second embodiment.
FIG. 4B is a cross-sectional view along line B-B of FIG. 4A.
FIG. 5A is a plan view of a cultivation container according to a third embodiment.
FIG. 5B is a cross-sectional view along line C-C of FIG. 5A.
FIG. 6A is a plan view of a cultivation container according to a fourth embodiment.
FIG. 6B is a cross-sectional view along line D-D of FIG. 6A.
FIG. 7 is a plan view of a cultivation container according to a fifth embodiment.
FIG. 8 is an exploded perspective view of a cultivation container according to a sixth embodiment.
FIG. 9 is a plan view of a cultivation container according to a seventh embodiment.
FIG. 10 is an exploded perspective view of a cultivation container according to an eighth embodiment.
FIG. 11 is a schematic view showing a schematic configuration of an observation system according to a ninth embodiment.
FIG. 12 is a schematic view showing a schematic configuration of an observation system according to a tenth embodiment.

### DETAILED DESCRIPTION OF THE INVENTION

Embodiments of the present invention will be described herein with reference to the drawings.

One or more embodiments provide a cultivation container and an observation system capable of observing a high contrast cell using an optical system.

Hereinafter, a cultivation container and an observation system according one or more embodiments will be described with reference to the accompanying drawings.

One or more embodiments relate to a cultivation container and an observation system capable of observing a high contrast cell using an optical system by making adherent cells glow locally. That is, one or more embodiments include a waveguide substrate configured toguide the measurement light while totally reflecting the light, and are carried out by making the adherent cell glow locally such that some of the measurement light locally enters the adherent cells in a cell cultivation space. The adherent cells cultivated in the cell cultivation space have a refractive index that is closer to that of a waveguide substrate in comparison with the case of air that is a gas and a cultivation liquid that is a liquid. For this reason, when the adherent cells are present in the cell cultivation space, some of the measurement light guided into the waveguide substrate enters the adherent cells, and the measurement light is scattered in an interface between the adherent cell and the waveguide substrate, and in the adherent cell. Accordingly, the adherent cells glow locally.

When the adherent cells in the cultivation container are observed during cultivation, in observation in a bright field in which light passing through an object is observed, a contrast is decreased. For this reason, in observation of the adherent cells in the bright field, a boundary between the cells becomes unclear, which is not suitable for observing unstained cells. A phase contrast microscope is one method of observing such adherent cells with good contrast, and includes a phase contrast optical system having a phase film and a dimmer filter. However, in the phase contrast microscope, since an effect of a phase contrast is weakened or cannot be obtained in an edge portion of the solution of the cultivation container, a region where a phase contrast image with good contrast can be obtained is limited to the central portion of the container.

Japanese Unexamined Patent Application Publication No. 2011-248216 and Japanese Unexamined Patent Application Publication No. 2018-081134 disclose a dark field optical system as a method of obtaining a high contrast image of an object. The dark field optical system is a technique of obtaining an image by causing measurement light to be incident on an observation object in an oblique direction with respect to an observation direction of a light receiving system and observing scattered light in the observation object. According to such a dark field optical system, it is possible to observe the adherent cell that is an object with good contrast regardless of the phase contrast image. However, in the case in which the dark field optical system is applied to observation of a cell image in the cultivation container, when the measurement light entering the cultivation container in the oblique direction is transferred into the cultivation container and spreads over the entire cultivation container, the measurement light leaks from various areas of the cultivation container. Such leakage light may interfere with observation.

In one or more embodiments, a waveguide substrate configured to totally reflect and guide the measurement light that enters it from side end surfaces, and a surrounding wall standing upright on a top surface of the waveguide substrate and forming a cell cultivation space therein are provided. Accordingly, the measurement light is refracted and enters the cell cultivation space from the waveguide substrate to be scattered in the cell locally at a place where the adherent cells adhere to the waveguide substrate. As a result, according to the one or more embodiments, it is possible to observe adherent cell with good contrast using an optical device such as a microscope, a camera with an equal magnification, or the like.

### [First embodiment]

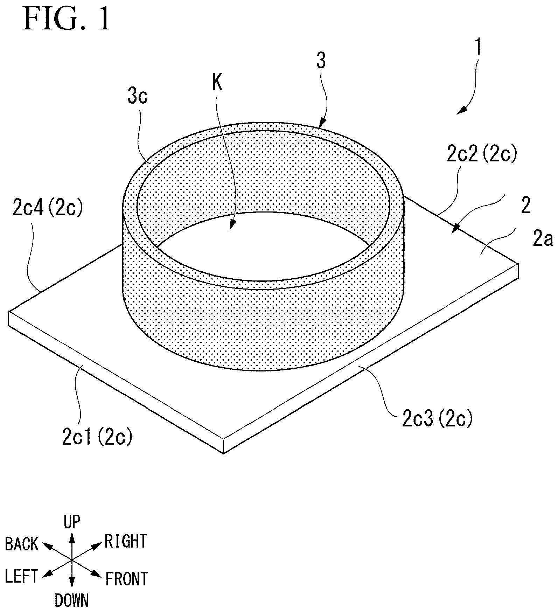

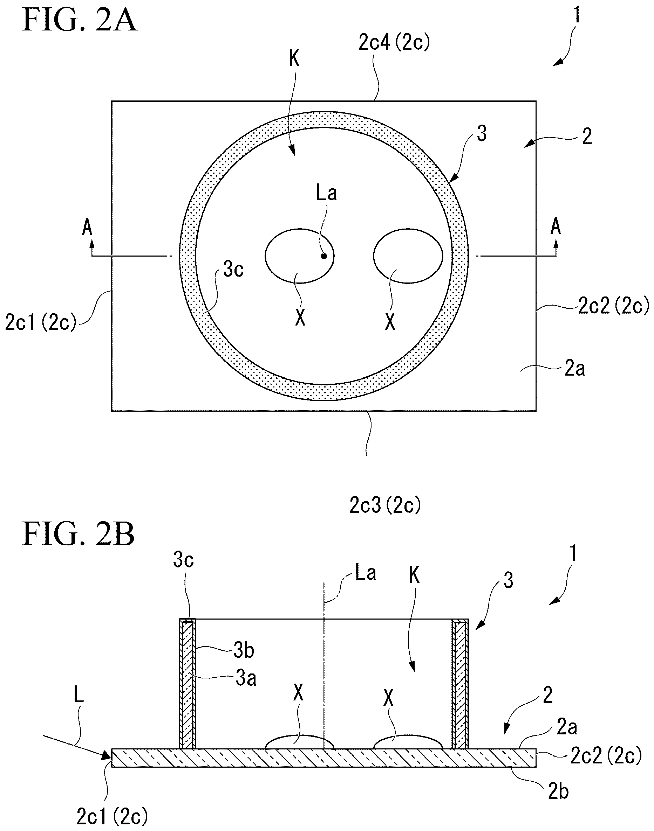

FIG. 1 is a perspective view of a cultivation container 1 of a first embodiment. In addition, FIG. 2A is a plan view of the cultivation container 1 of the first embodiment, and FIG. 2B is a cross-sectional view along line A-A of FIG. 2A. Further, in the following description, for the convenience of description, a direction in which a surrounding wall 3 protrudes from a waveguide substrate 2 is an upward direction. However, in a placement posture of the cultivation container 1 upon storage or the like, the upward direction set for the convenience of description does not necessarily coincide with an upward direction with respect to gravity.

The cultivation container 1 according to one or more embodiments is a container configured to perform cultivation of adherent cells X (cells that proliferate while adhering to the cultivation container 1), and it is possible to perform observation of the high contrast adherent cells X by introducing measurement light into the waveguide substrate 2 and using a light receiving system such as a camera or the like. As shown in FIG. 1, FIG. 2A, and FIG. 2B, the cultivation container 1 includes the waveguide substrate 2 and the surrounding wall 3.

The waveguide substrate 2 is a plate-shaped member in which an upper surface 2a (a top surface) and a bottom surface 2b are disposed to face each other in parallel. The waveguide substrate 2 in one or more embodiments has a rectangular shape in a plan view. Further, a direction along a long side of the waveguide substrate 2 in a plan view is referred to as a leftward/rightward direction for the convenience of description, and a direction along a short side of the waveguide substrate 2 in a plan view is referred to as a forward/rearward direction for the convenience of description.

In one or more embodiments, since a shape of the waveguide substrate 2 in a plan view is a rectangular shape, the waveguide substrate 2 has a left side end surface 2c1, a right side end surface 2c2, a front side end surface 2c3, and a rear side end surface 2c4, which are side end surfaces 2c. Each of the side end surfaces 2c is a plane.

In one or more embodiments, the left side end surface 2c1 is an incident end surface on which measurement light L is incident from the outside. The left side end surface 2c1 may be flattened (a surface roughness reduced) to be the same as one or more of the other side end surfaces 2c (the right side end surface 2c2, the front side end surface 2c3, and the rear side end surface 2c4) in order to minimize scattering when the measurement light L is incident.

In addition, in one or more embodiments, the upper surface 2a and the bottom surface 2b of the waveguide substrate 2 are planes. However, any one or both of the upper surface 2a and the bottom surface 2b may also be a curved surface or a concavo-convex surface. The upper surface 2a of the waveguide substrate 2 is a surface to which the adherent cells X are adhered. Further, a space surrounded by the surrounding wall 3 is a cell cultivation space K, and the adherent cells X are adhered to a partial region of the upper surface 2a facing the cell cultivation space K. In addition, the upper surface 2a of the waveguide substrate 2 is a connecting surface of the surrounding wall 3. In one or more embodiments, the surrounding wall 3 is connected to the waveguide substrate 2 through adhesion or fusion.

The waveguide substrate 2 functions as a waveguide that guides the measurement light L incident from the left side end surface 2c1 that is an incident end surface toward the right side end surface 2c2 while being totally reflected therein. A laser beam is used as the measurement light L. When a dimension from the upper surface 2a to the bottom surface 2b of the waveguide substrate 2 (i.e., a thickness dimension) is a range in which the entire the measurement light L is able to be incident, the dimension can be set arbitrarily on the precondition that operability of the cultivation container 1 or strength required to support the surrounding wall 3 can be secured.

In addition, the measurement light L may be a line laser beam that spreads in a plane in order to widen a measurable range. For this reason, for example, a widthwise dimension of the cultivation container 1 in the forward/rearward direction may be greater than a line length of the measurement light L in the forward/rearward direction.

The waveguide substrate 2 that functions as a waveguide of the measurement light L in this way is formed of a transparent material with respect to the measurement light L, and for example, may be formed of glass or polystyrene. For example, a laser beam with a peak wavelength of 520 nm is used as the measurement light L. For this reason, the waveguide substrate 2 is formed of a material that can guide the measurement light L of the laser beam.

Further, a refractive index of the cell membrane of the adherent cell cultivated in the cultivation container 1 is about 1.46 to 1.6. On the other hand, a refractive index of the waveguide substrate 2 is closer to that of a cell membrane of the adherent cell than the cultivation liquid or air. For example, a refractive index when the waveguide substrate 2 is formed of glass is 1.52, and a refractive index when the waveguide substrate 2 is formed of polystyrene is 1.59.

The surrounding wall 3 is a cylindrical member centered on an axial center La, and stands upright on the upper surface 2a of the waveguide substrate 2. The surrounding wall 3 is oriented with respect to the waveguide substrate 2 such that the axial center La is parallel to a normal direction of the upper surface 2a of the waveguide substrate 2, a lower end is connected to the upper surface of the waveguide substrate 2, and an upper end is an open end.

The surrounding wall 3 according to one or more embodiments is formed separately from the waveguide substrate 2, and then connected to the waveguide substrate 2. The surrounding wall 3 is connected to the waveguide substrate 2 via an adhesive agent, or connected to the waveguide substrate 2 through welding. For this reason, the surrounding wall 3 can also be separated from the waveguide substrate 2. That is, in one or more embodiments, the surrounding wall 3 is detachably attached to the waveguide substrate 2. When the surrounding wall 3 is detachably attached to the waveguide substrate 2 in this way, for example, it is also possible to use the waveguide substrate 2 without providing the surrounding wall 3.

As shown in FIG. 2B, the surrounding wall 3 has a base part 3a and a shielding coating 3b (a shielding part). The base part 3a is formed of, for example, a resin, and may be formed of a different material from the waveguide substrate 2. The base part 3a is a main member that functions as a strength member of the surrounding wall 3, and connected to the waveguide substrate 2.

The shielding coating 3b is a membrane member capable of shielding the measurement light L, and provided by covering the top surface of the base part 3a. The shielding coating 3b prevents the measurement light L entering the base part 3a of the surrounding wall 3 from the waveguide substrate 2 from leaking to the cell cultivation space K from the surrounding wall 3 by shielding the measurement light L.

Further, in one or more embodiments, the shielding coating 3b is provided to cover the entire base part 3a except a connecting surface to the waveguide substrate 2. However, since it is only necessary to prevent the measurement light L entering the surrounding wall 3 from leaking from the surrounding wall 3 to the cell cultivation space K, the shielding coating 3b can be provided only on the inner wall surface of the surrounding wall 3.

In addition, a shielding part configured to prevent the measurement light L from entering the base part 3a may be provided between the base part 3a and the waveguide substrate 2. In this case, the shielding part prevents the measurement light L from entering the base part 3a from the waveguide substrate 2, and thus prevents the measurement light L from leaking from the surrounding wall 3 to the cell cultivation space K. For this reason, a configuration in which the shielding coating 3b is not provided may be possible by providing a shielding part between the base part 3a and the waveguide substrate 2.

In addition, the entire surrounding wall 3 may be formed of a colored resin or metal through which the measurement light L cannot pass, and may prevent the measurement light L from entering the base part 3a from the waveguide substrate 2. In this case, the surrounding wall 3 is not divided into the base part 3a and the shielding coating 3b as described above, and the surrounding wall 3 itself functions as a shielding part.

When the waveguide substrate 2 and the surrounding wall 3 are formed of different materials, for example, the waveguide substrate 2 and the surrounding wall 3 are separately formed and then joined to each other. In this case, for example, the shielding coating 3b is coated on the base part 3a that was previously formed, and then the surrounding wall 3 is connected to the waveguide substrate 2 by an adhesive agent, welding, or the like.

In addition, the base part 3a may be previously connected to the waveguide substrate 2, and then the shielding coating 3b may be formed on the top surface of the base part 3a. In this case, for example, the base part 3a may be integrally formed of the same material as the waveguide substrate 2, and then the shielding coating 3b may be formed on the top surface of the base part 3a.

In addition, in one or more embodiments, an upper end surface 3c of the surrounding wall 3 is parallel to the upper surface 2a and the bottom surface 2b of the waveguide substrate 2. For this reason, the upper end surface 3c of the surrounding wall 3 also becomes horizontal when a posture of the waveguide substrate 2 is set such that the upper surface 2a and the bottom surface 2b of the waveguide substrate 2 become horizontal. As a result, a lid configured to close the cell cultivation space K from above can be easily placed on the upper end surface 3c of the surrounding wall 3.

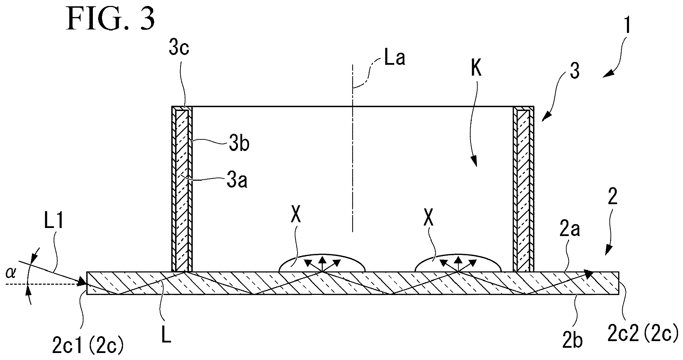

FIG. 3 is a schematic view when the measurement light L enters the cultivation container 1 of the above-mentioned embodiment. A difference between refractive indices of the waveguide substrate 2 and the air and cultivation liquid is great. For this reason, as shown in FIG. 3, the measurement light L entering the waveguide substrate 2 from the left side end surface 2c1 that is an incident end surface of the waveguide substrate 2 is guided toward the right side end surface 2c2 of the waveguide substrate 2 while being totally reflected by the upper surface 2a and the bottom surface 2b of the waveguide substrate 2.

In this way, the measurement light L advances while totally being reflected in the waveguide substrate 2. Here, when the adherent cells X are present in the cell cultivation space K surrounded by the surrounding wall 3, since the refractive index of the adherent cells X is closer to the waveguide substrate 2 than the air or cultivation liquid, some of the measurement light L enters the adherent cells X. The measurement light L entering the adherent cells X in this way is scattered in the adherent cells X and the interface between the adherent cells X and the waveguide substrate 2. As a result, when seen from above the adherent cells X, the measurement light L that advances through the waveguide substrate 2 is not visible, and the adherent cells X are locally visible.

Accordingly, the adherent cells X can be visually recognized with good contrast by observing the adherent cells X from above using the cultivation container 1 according to one or more embodiments. As a result, in the cultivation container 1 according to one or more embodiments, the adherent cells X can be observed with good contrast with a microscope using an optical system.

In addition, some of the measurement light L that advances through the waveguide substrate 2 enters the base part 3a of the surrounding wall 3. However, the measurement light L that enters the base part 3a of the surrounding wall 3 in this way is blocked by the shielding coating 3b. For this reason, it is possible to prevent some of the measurement light L from entering the cell cultivation space K from the surrounding wall 3.

Further, as the number of reflections of the measurement light L with the upper surface 2a in the waveguide substrate 2 is increased, chances of the measurement light L entering the adherent cells X can also be increased. For this reason, the number of reflections of the measurement light L may be large as long as the measurement light L does not deviate from the condition of total reflection. Accordingly, as shown FIG. 3, the measurement light L may be incident on the waveguide substrate 2 in a state in which an optical axis L1 is inclined with respect to the upper surface 2a and the bottom surface 2b of the waveguide substrate 2. For example, an angle α between the upper surface 2a and the bottom surface 2b and the optical axis L1 of the measurement light L may be about 4°.

The cultivation container 1 of the embodiment as described above includes the waveguide substrate 2 configured to totally reflect and guide the measurement light L incident from the left side end surface 2c1 thereinto, and the surrounding wall 3 standing on the upper surface 2a of the waveguide substrate 2 and forming the cell cultivation space K therein.

According to the cultivation container 1 of the above-mentioned embodiment, the measurement light L is guided while being totally reflected in the waveguide substrate 2. The refractive index of the adherent cells X cultivated in the cell cultivation space K is close to that of the waveguide substrate 2 when compared with air, which is a gas, or cultivation liquid, which is liquid. For this reason, when the adherent cells X are present in the cell cultivation space K, some of the measurement light L guided into the waveguide substrate 2 enters the adherent cells X, and the measurement light L is scattered in the interface between the adherent cells X and the waveguide substrate 2 or in the adherent cells X. Accordingly, it is possible to make the adherent cells X glow locally. As a result, in the cultivation container 1 according to one or more embodiments, it is possible to observe the high contrast adherent cells X using the optical system.

In addition, in the cultivation container 1 according to one or more embodiments, the surrounding wall 3 includes the shielding coating 3b that shields the measurement light L. According to the cultivation container 1 of the above-mentioned embodiment, it is possible to prevent some of the measurement light L from leaking from the surrounding wall 3 to the cell cultivation space K. Accordingly, it is possible to observe the adherent cells X of the cell cultivation space K with better contrast.

In addition, in the cultivation container 1 according to one or more embodiments, the surrounding wall 3 is formed of a material different from the waveguide substrate 2, and has the base part 3a detachably connected to the waveguide substrate 2 and the shielding coating 3b provided on the top surface of the base part 3a. According to the cultivation container 1 of the above-mentioned embodiment, since the surrounding wall 3 is separated from the waveguide substrate 2, selection of the material or the shape of the surrounding wall 3 can be designed separately from the waveguide substrate 2.

### [Second embodiment]

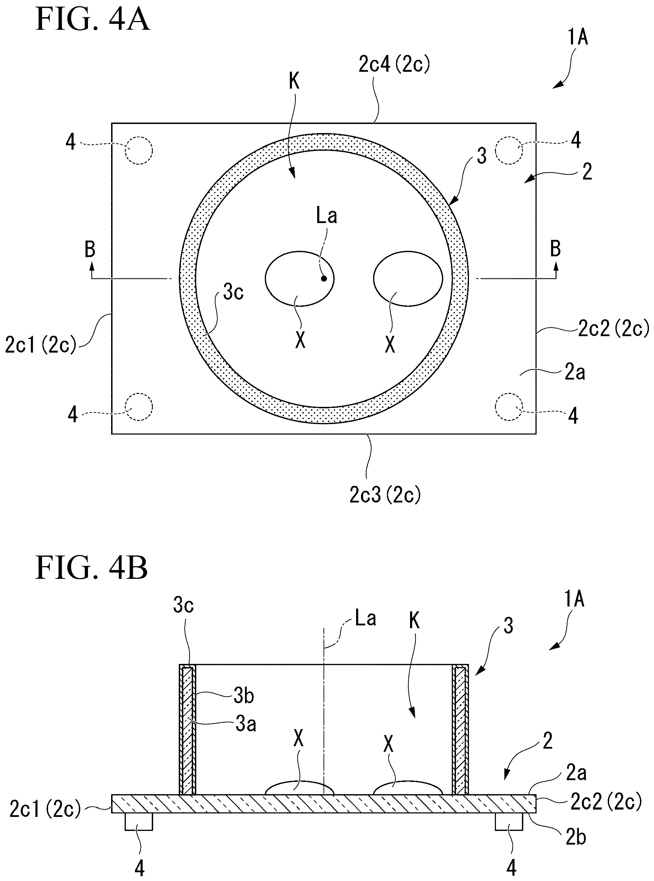

Next, a second embodiment will be described with reference to FIG. 4A and FIG. 4B. Further, in the description according to one or more embodiments, description of the same parts as in the first embodiment will be omitted or simplified.

FIG. 4A is a plan view of a cultivation container 1A according to one or more embodiments , and FIG. 4B is a cross-sectional view along line B-B of FIG. 4A. As shown in FIG. 4A and FIG. 4B, the cultivation container 1A according to one or more embodiments includes a plurality of support legs 4 distributed on the bottom surface 2b of the waveguide substrate 2. In one or more embodiments, a shape of the waveguide substrate 2 when seen in a plan view is a rectangular shape. The support legs 4 are provided at four corners of the waveguide substrate 2 having the rectangular shape when seen in a plan view.

Each of the support legs 4 protrudes downward from the bottom surface 2b of the waveguide substrate 2, and has a columnar shape in one or more embodiments. Further, the support leg 4 may be a prismatic, cylindrical, or rectangular tubular shape. When the cultivation container 1A according to one or more embodiments is installed on a floor surface, the support legs 4 directly abut the floor surface at lower surfaces thereof, and support the waveguide substrate 2 and the surrounding wall 3 from below. When the support legs 4 abut the floor surface, it is possible to prevent the bottom surface 2b of the waveguide substrate 2 and the floor surface from coming in contact with each other.

When the bottom surface 2b of the waveguide substrate 2 and the floor surface are in contact with each other, if the material forming the floor surface has a refractive index close to that of the waveguide substrate 2, the measurement light L leaks from the bottom surface 2b of the waveguide substrate 2 to the member forming the floor surface, and the efficiency of utilization of light may be reduced. On the other hand, according to the cultivation container 1A according to one or more embodiments, since the bottom surface 2b of the waveguide substrate 2 can be prevented from coming into contact with other members, it is possible to minimize leakage of the measurement light L from the bottom surface 2b of the waveguide substrate 2.

Downward protrusion amounts of the support legs 4 from the bottom surface 2b of the waveguide substrate 2 are the same as each other. For this reason, when all the support legs 4 cause the bottom surface 2b to abut the horizontal floor surface, the waveguide substrate 2 can be disposed such that the upper surface 2a and the bottom surface 2b are horizontal.

For example, the support legs 4 may be formed of a colored resin into which the measurement light L does not enter. In addition, the support legs 4 may also be formed of a transparent material into which the measurement light L enters. In this case, since the measurement light L leaked from the support legs 4 is prevented from becoming stray light that obstructs observation of the adherent cells X, the top surface of the support legs 4 may be covered with the coating that shields the measurement light L.

As described above, the cultivation container 1A according to one or more embodimentsincludes the plurality of support legs 4 distributed on the bottom surface 2b of the waveguide substrate 2 and configured to support the waveguide substrate 2 from below. For this reason, it is possible to prevent leakage of the measurement light L to the other member as the bottom surface 2b of the waveguide substrate 2 comes into contact with the other member. For this reason, in the cultivation container 1A according to one or more embodiments, it is possible to prevent the observation of the adherent cells X from being hindered by the leakage of the measurement light L, and to prevent the efficiency of utilization of the measurement light L from being decreased by the leakage of the measurement light L.

### [Third embodiment]

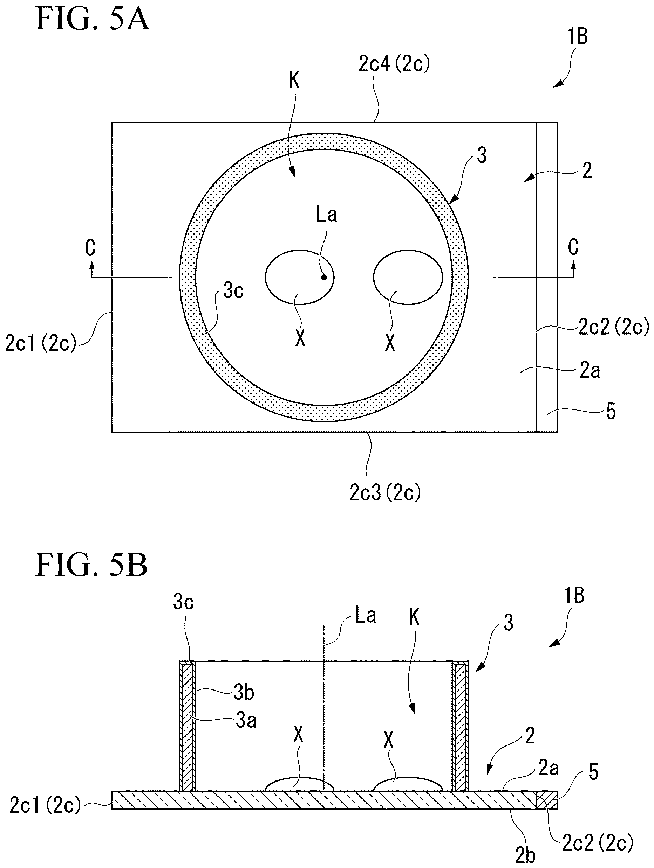

Next, a third embodiment will be described with reference to FIG. 5A and FIG. 5B. Further, in the description according to one or more embodiments, description of the same parts as in the first embodiment will be omitted or simplified.

FIG. 5A is a plan view of a cultivation container 1B according to one or more embodiments, and FIG. 5B is a cross-sectional view along line C-C of FIG. 5A. As shown in FIG. 5A and FIG. 5B, in the cultivation container 1B according to one or more embodiments, a reflecting plate 5 that abuts a right side end surface 2c2 of the waveguide substrate 2 from the outside is provided. The reflecting plate 5 may be referred to as a reflector or a reflecting part.

The reflecting plate 5 is a member having a mirror surface capable of reflecting the measurement light L, which reflects the measurement light L guided in the waveguide substrate 2 and exit the waveguide substrate 2 from the right side end surface 2c2 of the waveguide substrate 2 to return the measurement light L into the waveguide substrate 2. In one or more embodiments, the reflecting plate 5 is provided to cover the entire right side end surface 2c2 facing a left side end surface 2c1 that is an incident end surface of the waveguide substrate 2. However, the reflecting plate 5 may be provided to cover a front side end surface 2c3 or a rear side end surface 2c4.

In addition, in the above-mentioned cultivation container 1B according to one or more embodiments, the measurement light L reflected by the reflecting plate 5 is guided through the waveguide substrate 2, and some of the measurement light L is incident on the adherent cells X and scattered. For this reason, in order to avoid stray light in the waveguide substrate 2, the right side end surface 2c2 and the reflecting surface of the reflecting plate 5 may be as smooth as the left side end surface 2c1, which is the incident end surface.

In addition, when the reflecting plate 5 is installed in this way, the measurement light L reflected by the reflecting plate 5 and the measurement light L incident from the left side end surface 2c 1 that is the incident end surface are guided in the same waveguide substrate 2. For this reason, a length dimension of the waveguide substrate 2 in the leftward/rightward direction may be set by the measurement light L reflected by the reflecting plate 5 and the measurement light L incident from the left side end surface 2c1 that is the incident end surface such that an interference fringe does not occur in the waveguide substrate 2.

As described above, the cultivation container 1B according to one or more embodimentsincludes the reflecting plate 5 provided at a position different from a position on which the measurement light L is incident (in one or more embodiments, the left side end surface 2c1) and configured to reflect the measurement light L. For this reason, when the reflecting plate 5 is provided, the measurement light L that has passed through the waveguide substrate 2 can be returned to the waveguide substrate 2 and used for observation of the adherent cells X. As a result, according to the cultivation container 1B according to one or more embodiments, efficiency of utilization of the measurement light L can be increased.

Further, in one or more embodiments, the configuration including the plate-shaped reflecting plate 5 has been described. However, a reflecting part (reflector) having another shape may be installed instead of the reflecting plate 5. For example, a reflecting part formed of a block body may also be provided to abut the right side end surface 2c2 and cover the right side end surface 2c2.

### [Fourth embodiment]

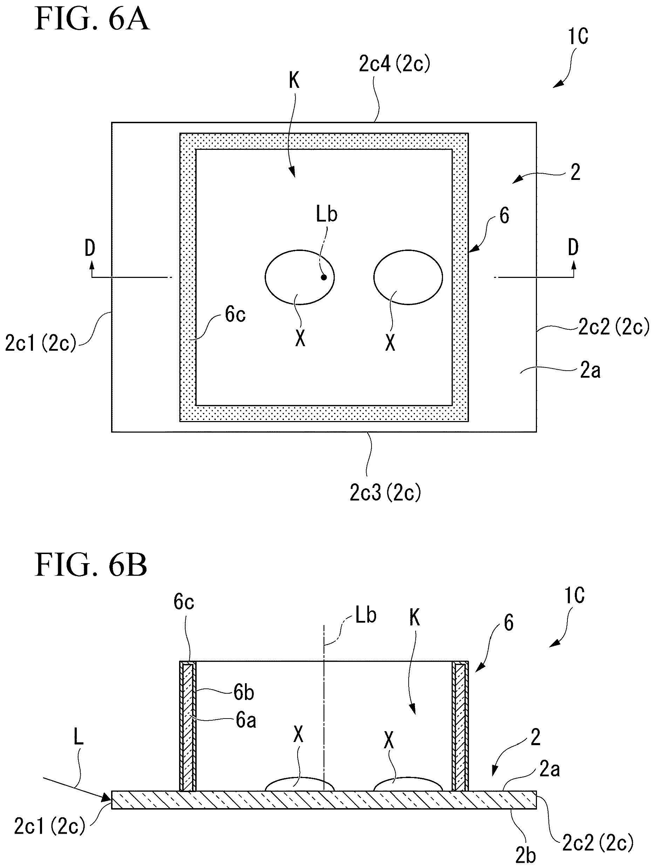

Next, a fourth embodiment will be described with reference to FIG. 6A and FIG. 6B. Further, in the description according to one or more embodiments, description of the same parts as in the first embodiment will be omitted or simplified.

FIG. 6A is a plan view of a cultivation container 1C according to one or more embodiments, and FIG. 6B is a cross-sectional view along line D-D of FIG. 6A. As shown in these drawing, in one or more embodiments, a surrounding wall 6 having a rectangular shape when seen in a plan view is provided.

The surrounding wall 6 is a rectangular tubular member centered on the axial center La, and stands upright on the upper surface 2a of the waveguide substrate 2. The posture of the surrounding wall 6 is set with respect to the waveguide substrate 2 such that an axial center Lb is parallel to a normal direction of the upper surface 2a of the waveguide substrate 2, and the surrounding wall 6 has a lower end that is connected to the upper surface of the waveguide substrate 2, and an upper end that is an open end.

The surrounding wall 6 according to one or more embodiments has a rectangular shape when seen in a plan view. One side in the rectangular shape is parallel to the side end surface 2c of the waveguide substrate 2. For example, one side of the surrounding wall 6 on the left side is parallel to the left side end surface 2c1 of the waveguide substrate 2. In addition, one side of the surrounding wall 6 on the right side is parallel to the right side end surface 2c2 of the waveguide substrate 2. In addition, one side of the surrounding wall 6 on the front side is parallel to the front side end surface 2c3 of the waveguide substrate 2. In addition, one side of the surrounding wall 6 on the rear side is parallel to the rear side end surface 2c4 of the waveguide substrate 2. However, the above-mentioned sides of the surrounding wall 6 may be inclined with respect to the side end surfaces 2c of the waveguide substrate 2.

The above-mentioned surrounding wall 6 is formed separately from the waveguide substrate 2, and then, connected to the waveguide substrate 2. The above-mentioned surrounding wall 6 is connected to the waveguide substrate 2 via an adhesive agent or connected to the waveguide substrate 2 through welding. For this reason, the surrounding wall 6 may be exfoliated from the waveguide substrate 2. That is, in one or more embodiments, the surrounding wall 6 is detachably attached to the waveguide substrate 2. When the surrounding wall 6 is detachably attached to the waveguide substrate 2 in this way, for example, the waveguide substrate 2 can also be used without providing the surrounding wall 6.

As shown in FIG. 6B, the surrounding wall 6 has a base part 6a and a shielding coating 6b (a shielding part). The base part 6a is formed of, for example, a resin, and may be formed of a material different from the waveguide substrate 2. The above-mentioned base part 6a is a main member that functions as a strength member of the surrounding wall 6, and connected to the waveguide substrate 2.

The shielding coating 6b is a membrane member capable of shielding the measurement light L, and provided by covering the top surface of the base part 6a. The shielding coating 6b prevents the measurement light L entering the base part 6a of the surrounding wall 6 from the waveguide substrate 2 from leaking from the surrounding wall 6 to the cell cultivation space K by shielding the measurement light L.

Further, in one or more embodiments, the shielding coating 6b is provided to cover the entire base part 6a except a connecting surface to the waveguide substrate 2. However, since it is only necessary to prevent the measurement light L that has entered the surrounding wall 6 from leaking from the surrounding wall 6 to the cell cultivation space K, the shielding coating 6b may be provided only on the inner wall surface of the surrounding wall 6.

In addition, the shielding part configured to prevent the measurement light L from entering the base part 6a may be provided between the base part 6a and the waveguide substrate 2. In this case, the shielding part prevents the measurement light L from entering the base part 6a from the waveguide substrate 2, and thus, prevents the measurement light L from leaking from the surrounding wall 6 to the cell cultivation space K. For this reason, a configuration in which the above-mentioned shielding coating 6b is not provided may be possible by providing the shielding part between the base part 6a and the waveguide substrate 2.

In addition, the entire surrounding wall 6 may be formed of a colored resin or metal through which the measurement light L does not pass to prevent the measurement light L from entering the base part 6a from the waveguide substrate 2. In this case, the surrounding wall 6 is not divided into the base part 6a and the shielding coating 6b as described above, and the surrounding wall 6 itself functions as a shielding part.

When the waveguide substrate 2 and the surrounding wall 6 are formed of different materials, for example, the waveguide substrate 2 and the surrounding wall 6 may be formed separately and then joined to each other. In this case, for example, the shielding coating 6b is formed on the base part 6a that was previously formed, and then, the surrounding wall 6 is connected to the waveguide substrate 2 by an adhesive agent, welding, or the like.

In addition, the base part 6a may be previously connected to the waveguide substrate 2, and then, the shielding coating 6b may be formed on the top surface of the base part 6a. In this case, for example, the base part 6a may be formed integrally with the waveguide substrate 2 by the same material, and then, the shielding coating 6b may be formed on the top surface of the base part 6a.

In addition, in one or more embodiments, an upper end surface 6c of the surrounding wall 6 is parallel to the upper surface 2a and the bottom surface 2b of the waveguide substrate 2. For this reason, the upper end surface 6c of the surrounding wall 6 is also horizontal by setting a posture of the waveguide substrate 2 such that the upper surface 2a and the bottom surface 2b of the waveguide substrate 2 are horizontal. As a result, a lid configured to close the cell cultivation space K from above can be easily placed on the upper end surface 6c of the surrounding wall 6.

In the cultivation container 1C according to one or more embodimentsas described above, a shape of the surrounding wall 6 when seen in a plan view is a rectangular shape. For this reason, when a maximum dimension in the leftward/rightward direction is the same as a maximum dimension in the forward/rearward direction, it is possible to secure a wide range of the cell cultivation space K in comparison with the surrounding wall having a circular shape when seen in a plan view.

### (Fifth embodiment)

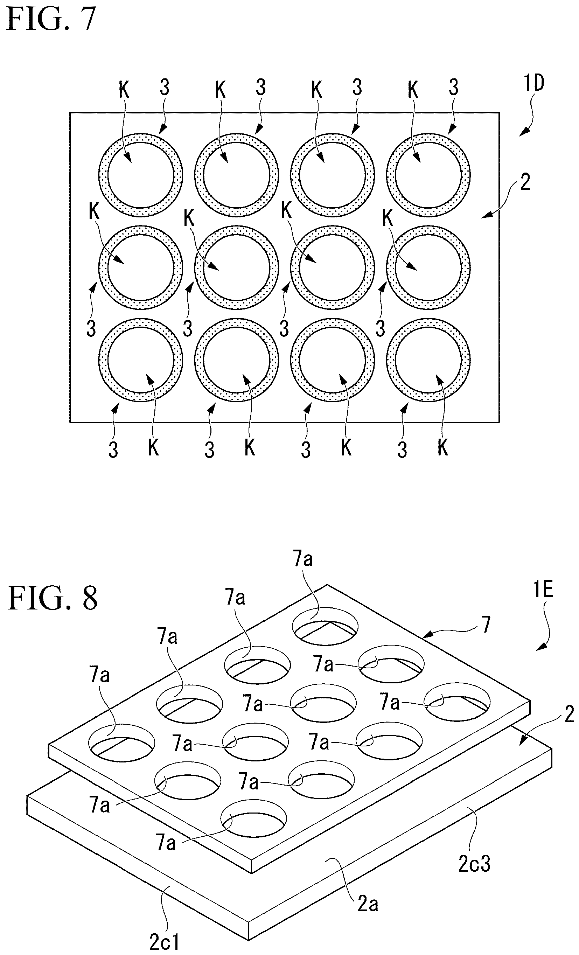

Next, a fifth embodiment will be described with reference to FIG. 7. Further, in the description according to one or more embodiments, description of the same parts as in the first embodiment will be omitted or simplified.

FIG. 7 is a plan view of a cultivation container 1D according to one or more embodiments. As shown in the drawing, in the cultivation container 1D according to one or more embodiments, the number of surrounding walls 3 are provided on the upper surface 2a of the waveguide substrate 2. The surrounding walls 3 are arranged in a lattice shape at equal intervals in the leftward/rightward direction and the forward/rearward direction.

In one or more embodiments, four surrounding walls 3 are arranged in the leftward/rightward direction, three surrounding walls 3 are arranged in the forward/rearward direction, and thus, the total twelve surrounding walls 3 are arranged on the upper surface 2a of the waveguide substrate 2. For this reason, the cultivation container 1D according to one or more embodimentshas 12 cell cultivation spaces K. Further, the number of the surrounding walls 3 is not limited to 12. For example, a configuration including 24 surrounding walls 3 may be employed.

For example, all the surrounding walls 3 have the same protrusion amount (i.e., height dimension) from the upper surface 2a of the waveguide substrate 2. For this reason, it is possible to close upper end openings of all the surrounding walls 3 with a single plate-shaped lid.

Further, in order to secure a capacity of each of the cell cultivation spaces K, when the plurality of surrounding walls 3 are provided like in the cultivation container 1D according to one or more embodiments, a size of the surrounding wall 3 is equal to that in the first embodiment, and a size of the waveguide substrate 2 is greater than that in the first embodiment.

In the cultivation container 1D of the above-mentioned embodiment, the plurality of surrounding walls 3 are provided on the single waveguide substrate 2. For this reason, the plurality of cell cultivation spaces K can be provided, and the adherent cells X can be divided and cultivated.

### [Sixth embodiment]

Next, a sixth embodiment will be described with reference to FIG. 8. Further, in the description according to one or more embodiments, description of the same parts as in the first embodiment will be omitted or simplified.

FIG. 8 is an exploded perspective view of a cultivation container 1E according to one or more embodiments. As shown in the drawing, the cultivation container 1E according to one or more embodimentsincludes a waveguide substrate 2 and a surrounding wall 7.

The surrounding wall 7 according to one or more embodimentshas a plurality of accommodating parts 7a having a circular shape when seen in a plan view. The accommodating parts 7a are arranged in a lattice shape at equal intervals in the leftward/rightward direction and the forward/rearward direction. In one or more embodiments, four accommodating parts 7a are arranged in the leftward/rightward direction, three accommodating parts 7a are arranged in the forward/rearward direction, and thus, the total 12 accommodating parts 7a are provided with respect to the surrounding wall 7. Further, the number of the accommodating parts 7a is not limited to 12. For example, a configuration including 24 accommodating parts 7a may be employed.

The accommodating parts 7a form the cell cultivation space K in a state in which the surrounding wall 7 is connected to the upper surface of the waveguide substrate 2. That is, the inside of the accommodating parts 7a is the cell cultivation space K. In this way, even one or more embodiments, the cell cultivation space K is formed in a part of the upper surface 2a of the waveguide substrate 2 by being surrounded by the surrounding wall 7.

For example, the surrounding wall 7 is formed of a material into which the measurement light L cannot enter. In this way, when the surrounding wall 7 is formed of the material into which the measurement light L cannot enter, it is possible to prevent the measurement light L from entering the surrounding wall 7 from the waveguide substrate 2 and prevent the light that interferes with observation of the adherent cells X from leaking from the surrounding wall 7.

Further, the surrounding wall 7 may be configured to include a base part that is a strength member, and a shielding coating configured to cover a top surface of the base part and shield the measurement light L. According to the above-mentioned configuration, even when some of the measurement light L enters the base part from the waveguide substrate 2, it is possible to prevent the measurement light that has entered the base part from leaking to the cell cultivation space K by being blocked by the shielding coating.

In the cultivation container 1E according to one or more embodiments as described above, the surrounding wall 7 includes the plurality of accommodating parts 7a that each forms the cell cultivation space K. For this reason, when the single surrounding wall 7 is adhered to the waveguide substrate 2, the number of cell cultivation spaces K can be formed at once.

### [Seventh embodiment]

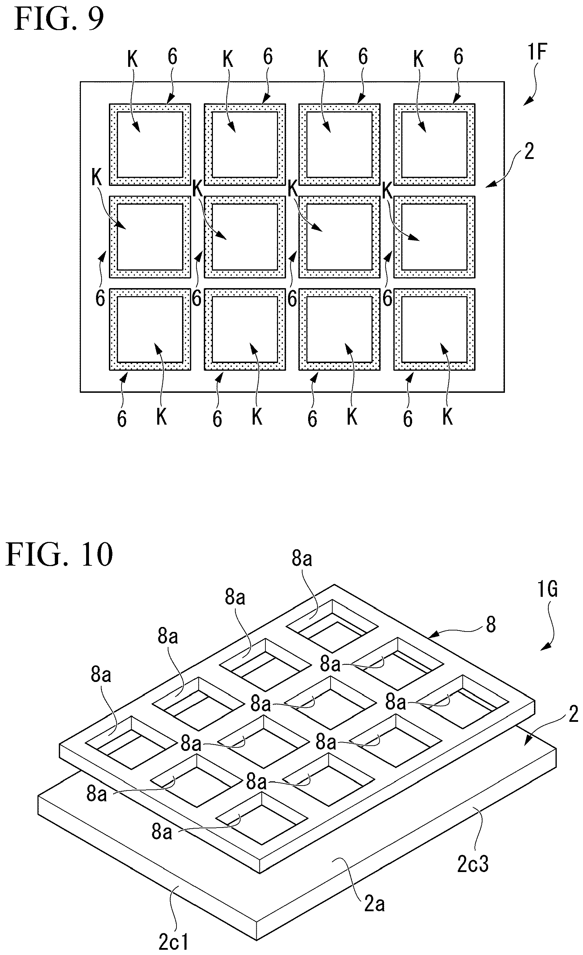

Next, a seventh embodiment will be described with reference to FIG. 9. Further, according to one or more embodiments, description of the same parts as in the first embodiment or the fourth embodiment will be omitted or simplified.

FIG. 9 is a plan view of a cultivation container IF according to one or more embodiments. As shown in the drawing, in the cultivation container IF according to one or more embodiments, the number of surrounding walls 6 are provided on the upper surface 2a of the waveguide substrate 2. The surrounding walls 6 are arranged in a lattice shape at equal intervals in the leftward/rightward direction and the forward/rearward direction.

In one or more embodiments, four surrounding walls 6 are arranged in the leftward/rightward direction, and three surrounding walls 6 are arranged in the forward/rearward direction. Thus, the total 12 surrounding walls 6 are provided on the upper surface 2a of the waveguide substrate 2. For this reason, the cultivation container IF according to one or more embodiments has 12 cell cultivation spaces K. Further, the number of the surrounding walls 6 is not limited to 12. For example, a configuration including 24 surrounding walls 6 may be employed.

For example, all the surrounding walls 6 have the same protrusion amount (i.e., height dimension) from the upper surface 2a of the waveguide substrate 2. For this reason, it is possible to close upper end openings of all the surrounding walls 6 with a single plate-shaped lid.

Further, in order to secure a capacity of each of the cell cultivation spaces K, when the plurality of surrounding walls 6 are provided like in the cultivation container IF according to one or more embodiments, a size of the surrounding wall 6 is the same as in the first embodiment, and a size of the waveguide substrate 2 is greater than that in the first embodiment.

In the cultivation container IF according to one or more embodiments as described above, the plurality of surrounding walls 6 are provided on the single waveguide substrate 2. For this reason, the plurality of cell cultivation spaces K can be provided, and the adherent cells X can be divided and cultivated.

In addition, a shape of the surrounding wall 6 when seen in a plan view is a rectangular shape. For this reason, when a maximum dimension in the leftward/rightward direction and a maximum dimension in the forward/rearward direction are the same, the cell cultivation space K can be widely secured in comparison with the surrounding wall having a circular shape when seen in a plan view. Accordingly, the cell cultivation spaces K can be widely secured on the upper surface 2a of the single waveguide substrate 2.

### [Eighth embodiment]

Next, an eighth embodiment will be described with reference to FIG. 10. Further, in the description according to one or more embodiments, description of the same parts as in the first embodiment will be omitted or simplified.

FIG. 10 is an exploded perspective view of a cultivation container 1G according to one or more embodiments. As shown in the drawing, the cultivation container 1G according to one or more embodiments includes a waveguide substrate 2 and a surrounding wall 8.

The surrounding wall 8 in one or more embodiments has a plurality of accommodating parts 8a having a rectangular shape when seen in a plan view. The accommodating parts 8a are arranged in a lattice shape at equal intervals in the leftward/rightward direction and the forward/rearward direction. In one or more embodiments, four accommodating parts 8a are arranged in the leftward/rightward direction, three accommodating parts 8a are arranged in the forward/rearward direction, and thus, the total 12 accommodating parts 8a are provided with respect to the surrounding wall 8. Further, the number of the accommodating parts 8a is not limited to 12. For example, a configuration including 24 accommodating parts 8a may be employed.

The accommodating parts 8a form the cell cultivation space K in a state in which the surrounding wall 8 is connected to the upper surface of the waveguide substrate 2. That is, the inside of the accommodating parts 8a is the cell cultivation space K. In this way, even in one or more embodiments, the cell cultivation space K is formed on a part of the upper surface 2a of the waveguide substrate 2 by being surrounded by the surrounding wall 8.

For example, the surrounding wall 8 is formed of a material into which the measurement light L cannot enter. When the surrounding wall 8 is formed of the material into which the measurement light L cannot enter in this way, it is possible to prevent the measurement light L from entering the surrounding wall 8 from the waveguide substrate 2 and prevent the light that interferes with observation of the adherent cells X from leaking from the surrounding wall 8.

Further, the surrounding wall 8 may be configured to include a base part that is a strength member, and a shielding coating configured to cover a top surface of the base part and configured to shield the measurement light L. According to the above-mentioned configuration, even when some of the measurement light L enters the base part from the waveguide substrate 2, it is possible to prevent the measurement light that has entered the base part from leaking to the cell cultivation space K by being shielded by the shielding coating.

In the cultivation container 1G according to one or more embodiments as described above, the surrounding wall 8 includes the plurality of accommodating parts 8a that each forms the cell cultivation space K. For this reason, when the single the surrounding wall 7 is adhered to the waveguide substrate 2, the number of cell cultivation spaces K can be formed at once.

In addition, in the cultivation container 1G according to one or more embodiments, a shape of each of the accommodating parts 8a when seen in a plan view is a rectangular shape. For this reason, in comparison with the accommodating part having a circular shape when seen in a plan view in which the maximum dimension in the leftward/rightward direction and the maximum dimension in the forward/rearward direction are the same, the cell cultivation space K can be widely secured. Accordingly, the cell cultivation space K can be widely secured on the upper surface 2a of the single waveguide substrate 2.

### (Ninth embodiment)

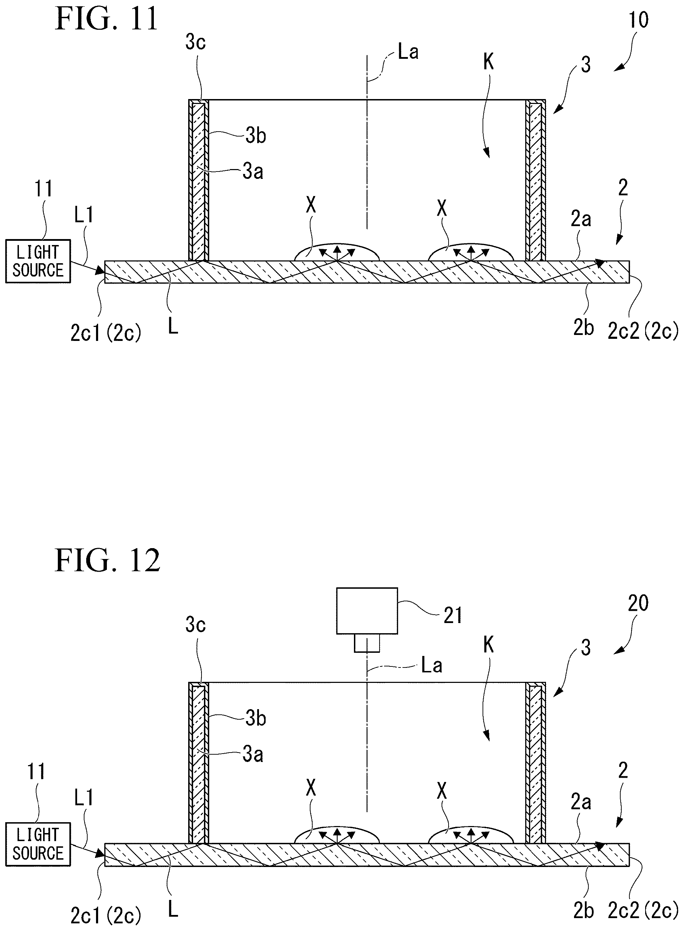

Next, a ninth embodiment will be described with reference to FIG. 11. Further, in the description according to one or more embodiments, description of the same parts as in the first embodiment will be omitted or simplified.

FIG. 11 is a schematic view showing a schematic configuration of an observation system 10 according to one or more embodiments. As shown in the drawing, the observation system 10 according to one or more embodiments includes a cultivation container 1 and a light source 11.

The light source 11 is a laser beam emitting device configured to emit the measurement light L. As shown in FIG. 11, the light source 11 is disposed aside the cultivation container 1 to face the left side end surface 2c1 of the waveguide substrate 2. The above-mentioned light source 11 emits the measurement light L such that the optical axis L1 of the measurement light L is inclined with respect to the upper surface 2a and the bottom surface 2b of the waveguide substrate 2 within a range that satisfies a condition in which the measurement light L is totally reflected in the waveguide substrate 2.

In the observation system 10 according to one or more embodiments as described above, the light source 11 configured to emit the measurement light L is provided. An output or the like with respect to the light source 11 can be previously set such that the measurement light L appropriate for observation of the adherent cells X is emitted. For this reason, adjustment of the measurement light L can be completed for a short time.

In addition, in the observation system 10 according to one or more embodiments, the upper surface 2a and the bottom surface 2b of the waveguide substrate 2 of the cultivation container 1 are planar, and the light source 11 is disposed such that the optical axis L1 of the measurement light L incident on the left side end surface 2c1 of the waveguide substrate 2 is inclined with respect to the upper surface 2a and the bottom surface 2b of the waveguide substrate 2. For this reason, the number of reflections of the measurement light L with the upper surface 2a in the waveguide substrate 2 can be increased, and chances of causing the measurement light L to be incident on the adherent cells X can be increased.

In addition, in the observation system 10 according to one or more embodiments, for example, a stage configured to define an installation position of the cultivation container 1 may be provided, and a configuration of directly or indirectly fixing the light source 11 to the stage may be possible. By employing the above-mentioned configuration, the cultivation container 1 can be easily placed at a position that is defined in advance, and further, a posture of the light source 11 with respect to the placed cultivation container 1 can be accurately determined as a posture appropriate for observation. For this reason, for example, the measurement light L can be reliably emitted such that the optical axis L1 forms a desired inclination angle with respect to the upper surface 2a and the bottom surface 2b of the waveguide substrate 2 of the cultivation container 1.

Further, in one or more embodiments, the configuration including the cultivation container 1 has been described. However, one or more embodiments are not limited thereto. A configuration including the cultivation container 1A of the second embodiment, the cultivation container 1B of the third embodiment, the cultivation container 1C of the fourth embodiment, the cultivation container 1D of the fifth embodiment, the cultivation container 1E of the sixth embodiment, the cultivation container IF of the seventh embodiment, or the cultivation container 1G of the eighth embodiment, instead of the cultivation container 1, may be employed.

### (Tenth embodiment)

Next, a tenth embodiment will be described with reference to FIG. 12. Further, in the description according to one or more embodiments, description of the same parts as in the ninth embodiment will be omitted or simplified.

FIG. 12 is a schematic view showing a schematic configuration of an observation system 20 according to one or more embodiments. As shown in the drawing, the observation system 20 according to one or more embodiments includes a cultivation container 1, a light source 11, and an optical system 21 (an observation part).

The optical system 21 is an optical system that can enlarge and observe the entirety or a part of the cell cultivation space K, and is disposed above the cultivation container 1. The optical system 21 receives the measurement light L dispersed in the adherent cells X, and enables observation by observer or imaging by a camera or the like due to formation of an image. Further, the optical system 21 is not limited in the installation position or the imaging direction, with the precondition that the adherent cells X in the cultivation container 1 can be observed. That is, the optical system 21 can be installed at various positions with respect to the cultivation container 1 and can image the adherent cells X in various directions.

In this way, the observation system 20 according to one or more embodiments includes the optical system 21. For this reason, it is possible to observe the adherent cells X cultivated in the cell cultivation space K without using an external optical system.

Hereinabove, while one or more embodiments have been described with reference to the accompanying drawings, it should be understood that the present invention is not limited to the above embodiments. All shapes, combinations, or the like, of each of components shown in the above-mentioned embodiments are exemplary, and various modifications may be made based on design requirements or the like without departing from the spirit of the present invention.

### [Supplementary Note]

(1) A cultivation container according to one or more embodiments employs a configuration including a waveguide substrate configured to totally reflect and guide measurement light incident from a side end surface of the waveguide substrate, and a surrounding wall standing upright on a top surface of the waveguide substrate and forming a cell cultivation space inside the surrounding wall.
(2) The cultivation container according to one or more embodiments employs a configuration in which the surrounding wall includes a shielding part configured to shield the measurement light.
(3) The cultivation container according to one or more embodiments employs a configuration in which the surrounding wall includes a base part formed of a material different from a material of the waveguide substrate and detachably attached to the waveguide substrate, and a shielding coating serving as the shielding part provided on a surface of the base part.
(4) The cultivation container according to one or more embodiments employs a configuration further including a plurality of support legs distributed on a bottom surface of the waveguide substrate and supporting the waveguide substrate from below.
(5) The cultivation container according to one or more embodiments employs a configuration further including a reflecting part (reflector) provided at a position different from a position of the side end surface on which the measurement light is incident and configured to reflect the measurement light.
(6) The cultivation container according to one or more embodiments employs a configuration in which a plurality of the surrounding wall is provided with respect to one waveguide substrate.
(7) The cultivation container according to one or more embodiments employs a configuration in which the surrounding wall includes a plurality of accommodating parts that form the cell cultivation space.
(8) The cultivation container according to one or more embodiments employs a configuration in which the side end surface to which the measurement light is incident is flattened to be the same as one or more of other side end surfaces of the waveguide substrate.
(9) The cultivation container according to one or more embodiments employs a configuration in which adherent cells are cultivated in the cell cultivation space, and a refractive index of the waveguide substrate is closer to a refractive index of a cell membrane of the adherent cells than cultivation liquid or air.
(10) An observation system according to one or more embodiments employs a configuration including the cultivation container according to (1), and a light source configured to emit the measurement light incident on the waveguide substrate of the cultivation container.
(11) The observation system according to one or more embodiments employs a configuration in which the top surface of the waveguide substrate of the cultivation container and a bottom surface facing the top surface of the waveguide substrate are planar, and the light source is disposed such that an optical axis of the measurement light incident on the side end surface is inclined with respect to the top surface and the bottom surface of the waveguide substrate.
(12) The observation system according to one or more embodiments employs a configuration including an observation part configured to observe the cell cultivation space.
(13) The observation system according to one or more embodiments employs a configuration in which the surrounding wall includes a shielding part configured to shield the measurement light.
(14) The observation system according to one or more embodiments employs a configuration in which the surrounding wall includes a base part formed of a material different from a material of the waveguide substrate and detachably attached to the waveguide substrate, and a shielding coating serving as the shielding part provided on a surface of the base part.
(15) The observation system according to one or more embodiments employs a configuration in which the cultivation container further includes a plurality of support legs distributed on a bottom surface of the waveguide substrate and supporting the waveguide substrate from below.
(16) The observation system according to one or more embodiments employs a configuration in which the cultivation container further includes a reflecting part provided at a position different from a position of the side end surface on which the measurement light is incident and configured to reflect the measurement light.
(17) The observation system according to one or more embodiments employs a configuration in which a plurality of the surrounding wall is provided with respect to one waveguide substrate.
(18) The observation system according to one or more embodiments employs a configuration in which the surrounding wall includes a plurality of accommodating parts that form the cell cultivation space.
(19) The observation system according to one or more embodiments employs a configuration in which he side end surface to which the measurement light is incident is flattened to be the same as one or more of other side end surfaces of the waveguide substrate.
(20) The observation system according to one or more embodiments employs a configuration in which adherent cells are cultivated in the cell cultivation space, and a refractive index of the waveguide substrate is closer to a refractive index of a cell membrane of the adherent cells than cultivation liquid or air.

According to one or more embodiments, measurement light is totally reflected and guided in the waveguide substrate. The adherent cells cultivated in the cell cultivation space have a refractive index close to that of the waveguide substrate in comparison with air that is a gas and a cultivation liquid that is a liquid. For this reason, when the adherent cells are present in the cell cultivation space, some of the measurement light guided in the waveguide substrate is refracted and incident in the adherent cells, and the measurement light is scattered in the interface between the adherent cells and the waveguide substrate or in the adherent cells. Accordingly, the adherent cells can locally glow. As a result, according to one or more embodiments, it is possible to observe high contrast cells using an optical system.

As used herein, the following directional terms "front, back, above, downward, right, left, vertical, horizontal, below, transverse, row and column" as well as any other similar directional terms refer to those instructions of a device according to one or more embodiments. Accordingly, these terms, as utilized to describe one or more embodiments should be interpreted relative to a device according to one or more embodiments.

The term "configured" is used to describe a component, unit or part of a device includes hardware and/or software that is constructed and/or programmed to carry out the function according to one or more embodiments.

Moreover, terms that are expressed as "means-plus function" in the claims should include any structure that can be utilized to carry out the function of that part of one or more embodiments.

The term "unit" is used to describe a component, unit or part of a hardware and/or software that is constructed and/or programmed to carry out the function according to one or more embodiments. Typical examples of the hardware may include, but are not limited to, a device and a circuit.

Although the disclosure has been described with respect to only a limited number of embodiments, those skilled in the art, having benefit of this disclosure, will appreciate that various other embodiments may be devised without departing from the scope of the present invention. Accordingly, the scope of the invention should be limited only by the attached claims.

## Claims

1. A cultivation container (1, 1A-1G)) comprising:
a waveguide substrate (2) configured to totally reflect and guide measurement light (L) incident from a side end surface (2c1) of the waveguide substrate (2); and
a surrounding wall (3, 6, 7, 8) standing upright on a top surface (2a) of the waveguide substrate (2) and forming a cell cultivation space (K).

2. The cultivation container (1) according to claim 1, wherein the surrounding wall (3) comprises a shielding part configured to shield the measurement light (L).

3. The cultivation container (1) according to claim 2, wherein:
the surrounding wall (3) comprises a base part (3a) formed of a material different from a material of the waveguide substrate (2) and detachably attached to the waveguide substrate (2); and
the shielding part comprises a shielding coating (3b) disposed on a surface of the base part (3a).

4. The cultivation container (1) according to any one of claims 1 to 3, further comprising:
support legs (4) distributed on a bottom surface (2b) of the waveguide substrate (2) and supporting the waveguide substrate (2) from below.

5. The cultivation container (1) according to any one of claims 1 to 4, further comprising:
a reflector (5) disposed at a position different from a position of the side end surface (2c1) on which the measurement light (L) is incident and configured to reflect the measurement light (L).

6. The cultivation container (1) according to any one of claims 1 to 5, further comprising at least one other surrounding wall (3, 6, 7, 8) standing upright on a top surface (2a) of the waveguide substrate (2) and forming a cell cultivation space (K).

7. The cultivation container (1) according to any one of claims 1 to 5, wherein the surrounding wall (3, 6, 7, 8) comprises accommodating parts (7a, 8a) that form the cell cultivation space (K).

8. The cultivation container (1) according to any one of claims 1 to 7, wherein the side end surface (2c1) to which the measurement light (L) is incident is flattened to be the same as one or more of other side end surfaces (2c2, 2c3, 2c4) of the waveguide substrate (2).

9. The cultivation container (1) according to any one of claims 1 to 8,
wherein adherent cells (X) are cultivated in the cell cultivation space (K), and
wherein a refractive index of the waveguide substrate (2) is closer to a refractive index of a cell membrane of the adherent cells (X) than cultivation liquid or air.

10. An observation system (10, 20) comprising:
the cultivation container (1) according to any one of claims 1 to 9; and
a light source (11) configured to emit the measurement light (L) incident on the waveguide substrate (2) of the cultivation container (1).

11. The observation system (10) according to claim 10,
wherein the top surface (2a) of the waveguide substrate (2) of the cultivation container (1) and a bottom surface (2b) facing the top surface (2a) of the waveguide substrate (2) are planar, and
wherein the light source (11) is disposed such that an optical axis (LI) of the measurement light (L) incident on the side end surface (2c1) is inclined with respect to the top surface (2a) and the bottom surface (2b) of the waveguide substrate (2).

12. The observation system (10) according to claim 10 or 11, further comprising:
an observation part (21) configured to observe the cell cultivation space (K).

13. The observation system (10) according to any one of claims 10 to 12,
wherein the surrounding wall (3) comprises a shielding part configured to shield the measurement light (L).

14. The observation system (10) according to claim 13, wherein:
the surrounding wall (3) comprises a base part (3a) formed of a material different from a material of the waveguide substrate (2) and detachably attached to the waveguide substrate (2); and
the shielding part comprises a shielding coating (3b) disposed on a surface of the base part (3a).

15. The observation system (10) according to any one of claims 10 to 14,
wherein the cultivation container (1) further comprises support legs (4) distributed on a bottom surface (2b) of the waveguide substrate (2) and supporting the waveguide substrate (2) from below.

16. The observation system (10) according to any one of claims 10 to 15, wherein the cultivation container (1) further comprises a reflector (5) disposed at a position different from a position of the side end surface (2c1) on which the measurement light (L) is incident and that reflects the measurement light (L).

17. The observation system (10) according to any one of claims 10 to 16, wherein the cultivation container (1) further comprises at least one other surrounding wall (3) standing upright on a top surface (2a) of the waveguide substrate (2) and forming a cell cultivation space (K).

18. The observation system (10) according to any one of claims 10 to 17, wherein the surrounding wall (3) comprises accommodating parts (7a, 8a) that form the cell cultivation space (K).

19. The observation system (10) according to any one of claims 10 to 18, wherein the side end surface (2c1) to which the measurement light (L) is incident is flattened to be the same as one or more of other side end surfaces (2c2, 2c3, 2c4) of the waveguide substrate (2).

20. The observation system (10) according to any one of claims 10 to 19,
wherein adherent cells (X) are cultivated in the cell cultivation space (K), and
wherein a refractive index of the waveguide substrate (2) is closer to a refractive index of a cell membrane of the adherent cells (X) than cultivation liquid or air.
